Europäisches Patentamt

European Patent Office

Office européen des brevets

(19)

(11) Publication number: **0 477 625 A1**

# EUROPEAN PATENT APPLICATION

(12)

(21) Application number: **91115077.9**

(22) Date of filing: **06.09.91**

(51) Int. Cl.5: **A61K 37/64**, //(A61K37/64, 31:435,31:505,31:415)

(30) Priority: **17.09.90 US 583749**

(43) Date of publication of application:
**01.04.92 Bulletin 92/14**

(84) Designated Contracting States:
**AT BE CH DE DK ES FR GB GR IT LI LU NL SE**

(71) Applicant: **E.R. SOUIBB & SONS, INC.**
**P.O.Box 4000**
**Princeton New Jersey 08543-4000(US)**

(72) Inventor: **Horovitz, Zola P.**
**30 Philip Drive**
**Princeton, NJ 08540(US)**

(74) Representative: **Josif, Albert et al**
**Baaderstrasse 3**
**W-8000 München 5(DE)**

(54) **Method for preventing or treating anxiety employing a combination of an ace inhibitor and a drug that acts at serotonin receptors.**

(57) A method is provided for inhibiting onset of or treating anxiety by administering an ACE inhibitor, such as captopril, fosinopril, zofenopril or ceranapril in combination with a drug that acts at serotonin receptors such as zacopride, over a prolonged period of treatment.

EP 0 477 625 A1

The present invention relates to a method for preventing or treating anxiety employing an angiotensin converting enzyme (ACE) inhibitor, such as captopril, SQ 29,852, zofenopril, fosinopril or enalapril, in combination with a drug that interacts with serotonin receptors in the brain, such as zacopride.

U. S. Patents Nos. 4,046,889 and 4,105,776 to Ondetti et al discloses proline derivatives, including captopril, which are angiotensin converting enzyme (ACE) inhibitors useful for treating hypertension.

U.S. Patent No. 4,337,201 to Petrillo discloses phosphinylalkanoyl substituted prolines, including fosinopril, which are ACE inhibitors useful for treating hypertension.

U.S. Patent No. 4,374,829 discloses carboxyalkyl dipeptide derivatives, including enalapril, which are ACE inhibitors useful for treating hypertension.

U.S. Patent No. 4,452,790 to Karanewsky et al, discloses phosphonate substituted amino or imino acids and salts thereof and covers (S)-1-[6-amino-2-[[hydroxy(4-phenylbutyl)-phosphinyl]oxy]-1-oxohexyl]-L-proline (ceranapril, SQ 29,852). These compounds are ACE inhibitors useful in treating hypertension.

U.S. Patent No. 4,316,960 to Ondetti et al, discloses ether and thioether mercaptoacyl prolines which are ACE inhibitors useful in treating hypertension. This Ondetti et al patent covers zofenopril.

U.S. Patent No. 4,931,430 to Sudilovsky et al, discloses a method for preventing or treating anxiety employing an ACE inhibitor in combination with a calcium channel blocker.

Angiotensin converting enzyme inhibitors and 5-HT$_3$ (5-hydroxytryptamine$_3$) receptor antagonists have previously been shown to improve the basal performance of laboratory animals in habituation paradigms and also to overcome the impairment induced by scopolamine (Costall et al (1989), "The effects of ACE inhibitors captopril and SQ29,852 in rodent tests of cognition." Pharmacol. Biochem. Behav. 33; 573-579; Barnes, J.M., et al (1990), "The effects of ondansetron, 5-HT$_3$ receptor antagonist, on cognition in rodents and primates." Pharmacol. Biochem. Behav. (35; 955-961).

Buspirone, a drug which interacts with serotonin receptors in the brain, is known for its use in treating anxiety (PDR, 1990, p. 1308).

Zacopride which also interacts with serotonin receptors in the brain is known for its use as an anti-emetic.

In accordance with the present invention, a method is provided for preventing or treating anxiety in mammalian species, over a prolonged period, wherein an antianxiety effective amount of a combination of an angiotensin converting enzyme inhibitor and a drug that interacts with serotonin receptors in the brain, is systemically, such as orally or parenterally, administered over a prolonged period, to prevent or treat anxiety during such period.

"Drugs that interact with serotonin receptors in the brain" and as such are employed in the method of the invention will hereinafter be referred to as "drugs that act at serotonin receptors." These drugs may or may not be 5-HT$_3$ antagonists.

The method of the invention is useful in treating or preventing anxiety including chronic and acute anxiety disorders (or anxiety and phobic neuroses) including panic disorder with or without agoraphobia, social phobia, simple phobia, obsessive compulsive disorder (or obsessive compulsive neurosis), post-traumatic stress disorder, generalized anxiety disorder, anxiety disorder not otherwise specified, and mixed anxiety-depression.

In addition, the method of the invention is useful in treating or preventing anxiety associated with withdrawal from drugs of dependency and/or addiction. Thus, the method of the invention is useful in reducing anxiety and thus facilitate withdrawal from alcohol dependency, nicotine dependency, cocaine dependency and benzodiazepine dependency as well as withdrawal from other drug dependency.

With respect to the combination of ACE inhibitor and a drug that acts at serotonin receptors in accordance with the present invention, the ACE inhibitor will be employed in a weight ratio to the drug that acts at serotonin receptors of within the range of from about 0.0005:1 to about $1 \times 10^7$:1 and preferably from about 0.002:1 to about $25 \times 10^4$:1.

The angiotensin converting enzyme inhibitor which may be employed herein includes substituted proline derivatives, such as any of those disclosed in U. S. Patent No. 4,046,889 or 4,105,776 to Ondetti et al mentioned above, with captopril, that is, 1-[(2S)-3-mercapto-2-methylpropionyl]-L-proline, being preferred, carboxyalkyl dipeptide derivatives, such as any of those disclosed in U.S. patent No. 4,374,829 mentioned above, with N-(1-ethoxycarbonyl-3-phenylpropyl)-L-alanyl-L-proline, that is, enalapril, being preferred.

Other examples of angiotensin converting enzyme inhibitors suitable for use herein include any of the phosphonate substituted amino or imino acids or salts disclosed in U. S. Patent No. 4,452,790 with (S)-1-[6-amino-2-[[hydroxy-(4-phenylbutyl)phosphinyl]oxy]-1-oxohexyl]-L-proline (ceranapril, SQ 29,852) being preferred, phosphinylalkanoyl prolines disclosed in U. S. Patent No. 4,168,267 mentioned above with fosinopril being preferred, mercaptoacyl derivatives of substituted prolines, disclosed in U. S. Patent No. 4,316,906 with zofenopril being preferred, any of the phosphinylalkanoyl substituted prolines disclosed in U. S. Patent No. 4,337,201 discussed above, and the phosphonamidates disclosed in U. S. Patent No. 4,432,971 discussed above.

Other examples of ACE inhibitors that may be employed herein include Beecham's BRL 36,378 as disclosed in European patent Nos. 80822 and 60668; Chugai's MC-838 disclosed in CA. 102:72588v and Jap. J. Pharmacol. 40:373 (1986); Ciba-Geigy's CGS 14824 (3-([1-ethoxycarbonyl-3-phenyl-(1S)-propyl]-amino)-2,3,4,5-tetrahydro-2-oxo-1-(3S)-benzazepine-1 acetic acid HCl) disclosed in U.K. Patent No. 2103614 and CGS 16,617 (3(S)-[[(1S)-5-amino-1-carboxypentyl]-amino]-2,3,4,5-tetrahydro-2-oxo-1H-1-benzazepine-1-ethanoic acid) disclosed in U. S. Patent No. 4,473,575; cetapril (alacepril, Dainippon) disclosed in Eur. Therap. Res. 39:671 (1986); 40:543 (1986); ramipril (Hoechst) disclosed in Eur. Patent No. 79-022 and Curr. Ther. Res. 40:74 (1986); Ru 44570 (Hoechst) disclosed in Arzneimittelforschung 35:1254 (1985), cilazapril (Hoffman-LaRoche) disclosed in J. Cardiovasc. Pharmacol. 9:39 (1987); $R_o$ 31-2201 (Hoffman-LaRoche) disclosed in FEBS Lett. 165:201 (1984); lisinopril (Merck) disclosed in Curr. Therap. Res. 37:342 (1985) and Eur. patent appl. No. 12-401, indalapril (delapril) disclosed in U. S. Patent No. 4,385,051; rentiapril (fentiapril, Santen) disclosed in Clin. Exp. Pharmacol. Physiol. 10:131 (1983); indolapril (Schering) disclosed in J. Cardiovasc. Pharmacol. 5:643, 655 (1983); spirapril (Schering) disclosed in Acta. Pharmacol. Toxicol. 59 (Supp. 5):173 (1986); perindopril (Servier) disclosed in Eur. J. Clin. Pharmacol. 31:519 (1987); quinapril (Warner-Lambert) disclosed in U. S. Patent No. 4,344,949 and CI 925 (Warner-Lambert) ([3S-[2[R(*)R(*)]]3R-(*)]-2-[2-[[1-(ethoxycarbonyl)-3-phenylpropyl]amino[-1-oxopropyl]-1,2,3,4-tetrahydro-6,7-dimethoxy-3-isoquinolinecarboxylic acid HCl) disclosed in Pharmacologist 26:243, 266 (1984), WY-44221 (Wyeth) disclosed in J. Med. Chem. 26:394 (1983).

Preferred are those ACE inhibitors which are proline or substituted proline derivatives, especially ceranapril, captopril, zofenopril and fosinopril .

The above-mentioned U.S. patents are incorporated herein by reference.

The drug that acts at serotonin receptors suitable for use herein may be zacopride (benzamide zacopride), $3\alpha$-tropanyl-IH-indole-3-carboxylic acid ester (ICS 205930, Sandoz); [endo]N-(9-methyl-9-azabicyclo-[3,3,I]-non-3-yl)-I-methyl-IH-indazole-3-carboxamide hydrochloride (BRL 43694, Granisetron, Beecham); ($I\alpha H,3\alpha,5\alpha H$-tropan-3-yl-3,5-dichlorobenzoate (MDL 72222, Merrell Dow); ondansetron; buspirone; and ritanserin.

A preferred combination in accordance with the present invention is ceranapril and zacopride.

In carrying out the method of the present invention, the angiotensin converting enzyme inhibitor in combination with the drug that acts at serotonin receptors may be administered to mammalian species, such as monkeys, dogs, cats, rats and humans, and as such may be incorporated in a conventional systemic dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid or sodium bisulfite) or the like. Oral dosage forms are preferred, although parenteral forms such as intramuscular, intraperitoneal, or intravenous are quite satisfactory as well.

The dose administered must be carefully adjusted according to age, weight and condition of the patient, as well as the route of administration, dosage form and regimen and the desired result.

Thus, for oral administration, a satisfactory result may be obtained employing the ACE inhibitor in an amount within the range of from about 0.005 mg/kg to about 100 mg/kg and preferably from about 0.01 mg/kg to about 25 mg/kg, in combination with the drug that acts at serotonin receptors in an amount within the range of from about 0.010 $\mu$g/kg to about 10 mg/kg and preferably from about 0.1 $\mu$g/kg to about 5 mg/kg, with the ACE inhibitor and drug that acts at serotonin receptors being employed together in the same oral dosage form or in separate oral dosage forms taken at the same time.

A preferred oral dosage form , such as tablets or capsules, will contain the ACE inhibitor in an amount of from about 0.1 to about 500 mg, preferably from about 5 to about 200 mg, and more preferably from about 25 to about 150 mg, with the drug that acts at serotonin receptors in an amount of from about 1 $\mu$g to about 200 mg, preferably from about 5 $\mu$g to about I50 mg, and more preferably from about I0 $\mu$g to about 100 mg.

For parenteral administration, the ACE inhibitor will be employed in an amount within the range of from about 0.005 mg/kg to about 10 mg/kg and preferably from about 0.01 mg/kg to about 1 mg/kg, and the drug that acts at serotonin receptors will be employed in an amount within the range of from about 0.005 $\mu$g/kg to about 20 mg/kg and preferably from about 0.01 $\mu$g/kg to about I0 mg/kg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 50 to 700 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonfuls.

Such dosage forms can be administered to the patient on a regimen of one to four doses per day.

According to another modification, in order to more finely regulate the dosage schedule, the active substances may be administered separately in individual dosage units at the same time or carefully coordinated times. Since blood levels are built up and maintained by a regulated schedule of administration, the same result is achieved by the simultaneous presence of the two substances. The respective substances can be individually formulated in separate unit dosage forms in a manner similar to that described above.

Fixed combinations of ACE inhibitor and drug that acts at serotonin receptors are more convenient and are preferred, especially in tablet or capsule form for oral administration.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Many of the active substances described above form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for as long as the potential for onset of anxiety continue. Sustained release forms of such formulations which may provide such amounts biweekly, weekly, monthly and the like may also be employed. A dosing period of at least one to two weeks are required to achieve minimal benefit.

The following Examples represent preferred embodiments of the present invention.

Example I

An injectable solution for use in treating anxiety is produced as follows:

| Ceranapril (SQ29,852) | 500 mg |
|---|---|
| Methyl paraben | 5 mg |
| Propyl paraben | l mg |
| Sodium chloride | 25 g |
| Water for injection qs. | 5 l. |

The SQ29,852, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 mL of solution in a concentration of 0.5 mg of active ingredient per mL of solution for injection.

A zacopride injectable solution for use in combination with ceranapril for treating anxiety is prepared as described above except l00 mg zacopride is employed in place of ceranapril.

The so-prepared injectable solutions may be administered separately or as a single injection to treat anxiety.

### Example 2

Two piece #l gelatin capsules each containing l00 mg of ceranapril are filled with a mixture of the following ingredients:

| Ceranapril | 100 mg |
|---|---|
| Zacopride | l mg |
| Magnesium stearate | 7 mg |
| USP lactose | l93 mg. |

The resulting capsules are useful in treating anxiety.

### Example 3

A captopril formulation suitable for oral administration for use in combination with a 5-HT$_3$ antagonist in treating anxiety is set out below.

1000 tablets each containing 100 mg of 1-[(2S)-3-mercapto-2-methylpropionyl]-L-proline were produced from the following ingredients.

| 1-[(2S)-3-Mercapto-2-methylpropionyl]-L-proline (captopril) | 100 g |
|---|---|
| Corn starch | 50 g |
| Gelatin | 7.5 g |
| Avicel (microcrystalline cellulose) | 25 g |
| Magnesium stearate | 2.5 g |

The captopril and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 0.5 mg of active ingredient which together with the drug that acts at serotonin receptors ICS 250930 (5-HT$_3$ antagonist) is used for treating anxiety.

The ICS 250930 (3$\alpha$-tropanyl-lH-indole-3-carboxylic acid ester) is formulated as l00 mg tablets as described above with respect to captopril.

The captopril tablets and ICS 250930 tablets may be administered to prevent onset of or treat anxiety.

### Example 4

By substituting 100 g of 1-(3-mercapto-2-D-methylpropanoyl)-L-proline for the captopril in Example 3 and adding l00 mg Granisetron, 1000 tablets each containing 100 mg of the 1-(3-mercapto-2-D-methylpropanoyl)-L-proline and l mg Granisetron are produced which are useful in treating or preventing onset of anxiety.

### Example 5

1000 tablets each containing 50 mg of fosinopril and 0.5 mg MDL 72222 (l$\alpha$H,3$\alpha$,5$\alpha$H-tropan-3-yl-3,5-dichlorobenzoate) are produced from the following ingredients:

| Fosinopril | 50 g |
|---|---|
| MDL 72222 | 0.5 g |
| Lactose | 100 g |
| Avicel | 150 g |
| Corn starch | 50 g |
| Magnesium stearate | 5 g |

The fosinopril, MDL 72222, lactose and Avicel are admixed, then blended with the corn starch. Magnesium stearate is added. The dry mixture is compressed in a tablet press to form 1000 505 mg tablets each containing 50.5 mg of active ingredients. The tablets are coated with a solution of Methocel E 15 (methyl cellulose) including as a color a lake containing yellow #6. The resulting tablets are useful in treating or preventing onset of anxiety.

Example 6

Two piece #1 gelatin capsules each containing l00 mg of enalapril and l00 mg zacopride are filled with a mixture of the following ingredients:

| Enalapril | 100 mg |
|---|---|
| Zacopride | 0.l mg |
| Magnesium stearate | 7 mg |
| USP lactose | 193 mg. |

The resulting capsules are useful in treating or preventing onset of anxiety.

**Claims**

1. Use of an angiotensin converting enzyme inhibitor with a drug that acts at serotonin receptors for preparing a medicine for inhibiting onset of or treating anxiety in a mammalian specie.

2. Use as defined in Claim 1 wherein the angiotensin converting enzyme inhibitor is a phosphonate substituted amino or imino acid or salt thereof, a proline derivative, a substituted proline derivative, a carboxyalkyl dipeptide derivative, a phosphinylalkanoyl proline derivative or a phosphonamidate derivative.

3. Use as defined in Claim 1 wherein said angiotensin converting enzyme inhibitor is a proline derivative or a substituted proline derivative.

4. Use as defined in Claim 1 wherein said angiotensin converting enzyme inhibitor is a carboxyalkyl dipeptide derivative.

5. Use as defined in Claim 1 wherein said angiotensin converting enzyme inhibitor is a phosphinylalkanoyl proline derivative, a phosphoramidate derivative, or a phosphonate substituted amino or imino acid or salt thereof.

6. Use as defined in Claim 1 wherein said angiotensin converting enzyme inhibitor is captopril.

7. Use as defined in Claim 1 wherein said angiotensin converting enzyme inhibitor is enalapril or lisinopril.

8. Use as defined in Claim 1 wherein said angiotensin converting enzyme inhibitor is zofenopril or fosinopril.

9. Use as defined in Claim 1 wherein said angiotensin converting enzyme inhibitor is (S)-l-[6-amino-2-[-[hydroxy(4-phenylbutyl)phosphinyl]-oxy]-l-oxohexyl]-L-proline (ceranapril).

10. Use as defined in Claim I wherein the drug that acts on serotonin receptors is zacopride; $3\alpha$-tropanyl-IH-indole-3-carboxylic acid ester; [endo]N-(9-methyl-9-azabicyclo-[3,3,l]-non-3-yl)-l-methyl-lH-indazole-3-carboxamide; l$\alpha$H,3$\alpha$,5$\alpha$H-tropan-3-yl-3,5-dichlorobenzoate; ondansetron; buspirone or ritanserin.

11. Use as defined in Claim I wherein the drug that acts at serotonin receptors is zacopride.

12. Use as defined in Claim I wherein the ACE inhibitor is ceranapril and the drug that acts at serotonin receptors is zacopride.

13. Use as defined in Claim 1 wherein said angiotensin converting enzyme inhibitor is administered in single or divided doses of from about 0.l to about 500 mg/one to four times daily and the drug that acts at serotonin receptors is administered in single or divided doses of from about l μg to about 200 mg/l to 4 times daily.

14. Use as defined in Claim 1 wherein the angiotensin converting enzyme inhibitor is employed in a weight ratio to the drug that acts at serotonin receptors of within the range of from about 0.0005:l to about lxl0$^7$:l.

15. Use of an angiotensin converting enzyme inhibitor alone or in combination with a drug that acts at serotonin receptors for preparing a medicine for inhibiting onset of or treating anxiety in a mammalian specie associated with withdrawal from drugs of dependency and/or addiction.

16. Use as defined in Claim l5 for reducing anxiety associated with nicotine withdrawal, alcohol withdrawal, diazepam withdrawal or cocaine withdrawal.

17. Use as defined in Claim l5 wherein the angiotensin converting enzyme inhibitor administered is captopril, SQ 29,852, fosinopril, zofenopril, enalapril or lisinopril and the drug that acts at serotonin receptors is zacopride; 3α-tropanyl-lH-indole-3-carboxylic acid ester; [endo]N-(9-methyl-9-azabicyclo-[3,3,l]-non-3-yl)-l-methyl-lH-indazole-3-carboxamide; lαH,3α,5αH-tropan-3-yl-3,5-dichlorobenzoate; ondansetron, buspirone; or ritanserin.

18. Use as defined in Claim l5 wherein the drug that acts at serotonin receptors is zacopride; 3α-tropanyl-lH-indole-3-carboxylic acid ester; [endo]N-(9-methyl-9-azabicyclo-[3,3,l]-non-3-yl)-l-methyl-lH-indazole-3-carboxamide; lαH,3α,5αH-tropan-3-yl-3,5-dichlorobenzoate; ondansetron; buspirone or ritanserin.

19. Use as defined in Claim l5 wherein the angiotensin converting enzyme inhibitor is ceranapril and the drug that acts at serotonin receptors is zacopride.

European Patent
Office

PARTIAL EUROPEAN SEARCH REPORT

which under Rule 45 of the European Patent Convention
shall be considered, for the purposes of subsequent
proceedings, as the European search report

Application Number

EP 91 11 5077

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | PHARMACOLOGY, vol. 38, no. 5, May 1989, pages 273-278, S. Karger Medical and Scientific Publishers, Basel, CH; B. MALINOWSKA et al.: "Influence of captopril on the vasopressor effect of serotonin in pithed rats" <br> * Results * <br> --- | 1-12,14 -19 | A 61 K   37/64  // <br> (A 61 K   37/64 <br> A 61 K   31:435 <br> A 61 K   31:505 <br> A 61 K   31:415) |
| A | CURR. THER. RES. CLIN. EXP., vol. 40, no. 5, November 1986, pages 917-923, US; M.J. NAPOLIELLO: "A study of buspirone coprescribed with antidepressants in 184 anxious ambulatory patients" <br> * Abstract * <br> --- | 1-12,14 -19 | |
| A | US-A-4 912 096  (E.R. SQUIB & SONS, INC.) <br> * Abstract * <br> ---                      -/- | 1-12,14 -19 | |

TECHNICAL FIELDS
SEARCHED (Int. Cl.5)

A 61 K

## INCOMPLETE SEARCH

The Search Division considers that the present European patent application does not comply with
the provisions of the European Patent Convention to such an extent that it is not possible to carry
out a meaningful search into the state of the art on the basis of some of the claims

Claims searched completely :    1-12,14-19

Claims searched incompletely :

Claims not searched :          13

Reason for the limitation of the search:

Method for treatment of the human or animal
body by surgery or therapy (see Art. 52(4)
of the European Patent Convention).

See also sheet -C-

| Place of search | Date of completion of the search | Examiner |
|---|---|---|
| THE HAGUE | 19-12-1991 | LEHERTE C.F.M. |

EPO FORM 1503 03.82 (P0407)

**European Patent Office**

**PARTIAL EUROPEAN SEARCH REPORT**

Application Number

EP 91 11 5077

| | DOCUMENTS CONSIDERED TO BE RELEVANT | | CLASSIFICATION OF THE APPLICATION (Int. Cl. 5) |
|---|---|---|---|
| Category | Citation of document with indication, where appropriate, of relevant passages | Relevant to claim | |
| X | EP-A-0 381 075 (E. R. SQUIBB & SONS, INC.)<br>* Claims 1-11,17 *<br>--- | 15-17 | |
| A | EP-A-0 381 074 (E. R. SQUIBB & SONS, INC.)<br>* Claims 1-11,17 *<br>--- | 15-19 | |
| A | EP-A-0 285 008 (BRISTOL-MYERS CO.)<br>* Claims 1-3 *<br>--- | 15-19 | |
| A | AM. J. PSYCHIATRY, vol. 136, no. 9, September 1979, pages 1184-1187, American Psychiatric Association; US; H.L. GOLDBERG et al.: "The comparative efficacy of buspirone and diazepam in the treatment of anxiety"<br>* Page 1186, column 2, line 27 - page 1187, column 1, line 15 *<br>----- | 1-12,14 -19 | |

**TECHNICAL FIELDS SEARCHED (Int. Cl.5)**

EPO FORM 1503 03.82 (P0410)

EP 91 11 5077  -C-

Claims searched completely: 12,19
Claims searched incompletely: 1-11,14-18

The definitions of compounds as an "ACE inhibitor
(which) is a phosphonate substituted amino or imino
acid or salt thereof" or as a derivative of an
other compound, or as a "drug that acts at serotonen
receptors" are not concise enough.
As a comsequence the search has been performed
for the compounds mentioned in the description.